(19) Europäisches Patentamt
European Patent Office
Office européen des brevets

(11) **EP 4 763 724 A1**

(12) **EUROPEAN PATENT APPLICATION**
published in accordance with Art. 153(4) EPC

(43) Date of publication:
**24.06.2026 Bulletin 2026/26**

(21) Application number: **24854050.2**

(22) Date of filing: **30.04.2024**

(51) International Patent Classification (IPC):
**B65B 55/10** (2006.01) **B65B 55/04** (2006.01)

(52) Cooperative Patent Classification (CPC):
**B65B 55/04; B65B 55/10**

(86) International application number:
**PCT/JP2024/016655**

(87) International publication number:
**WO 2025/037458 (20.02.2025 Gazette 2025/08)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **17.08.2023 JP 2023132904**

(71) Applicant: **Mitsubishi Heavy Industries Machinery
Systems, Ltd.
Kobe-shi, Hyogo 652-8585 (JP)**

(72) Inventors:
• **WATANABE, Atsunori
Kobe-shi, Hyogo 652-8585 (JP)**

• **YUI, Sugihiko
Kobe-shi, Hyogo 652-8585 (JP)**
• **TSUO, Atsushi
Kobe-shi, Hyogo 652-8585 (JP)**
• **SHIRASAYA, Kento
Kobe-shi, Hyogo 652-8585 (JP)**
• **KOMATSU, Yoshinao
Tokyo 100-8332 (JP)**
• **KAWANO, Takashi
Tokyo 100-8332 (JP)**
• **MIZUNO, Motohiro
Kobe-shi, Hyogo 652-8585 (JP)**

(74) Representative: **Hoffmann Eitle
Patent- und Rechtsanwälte PartmbB
Arabellastraße 30
81925 München (DE)**

(54) **STERILIZATION AGENT SUPPLY DEVICE, NOZZLE, AND STERILIZATION METHOD**

(57) This sterilization agent supply device comprises: a supply unit including a discharge tube via which a sterilization agent is supplied downward in the vertical direction at a fixed position; and a transport unit including a transport body that moves a plurality of objects being sterilized along a transport path while holding the plurality of objects being sterilized by using the holding tool, and moving nozzles that are provided to the transport body in correspondence with each of the plurality of objects being sterilized, the moving nozzles moving in synchronization with the corresponding objects being sterilized. The moving nozzles are each provided with a first flow path via which the sterilization agent is received on the upstream side and via which the sterilization agent is channeled toward the objects being sterilized on the downstream side. The first flow paths are each formed such that the opening area decreases continuously or intermittently from the upstream side to the downstream side.

FIG. 3

## Description

Technical Field

The present disclosure relates to a device for supplying a sterilization agent in a fluid state to, for example, a container of a beverage.

Background Art

In order to sterilize a container of a beverage, for example, a container made of plastic, a sterilization agent or a sterilant, for example, hydrogen peroxide ($H_2O_2$), is supplied from a discharge pipe or a discharge nozzle to an inside of the container. PTL 1 discloses a device including a sterilizing unit that sprays a sterilization agent in a fluid state, which is typically a mist, to the inside of the container.

The sterilizing unit in PTL 1 includes one or a plurality of discharge pipes that supply a condensed mist of hydrogen peroxide, which is a sterilization agent, to the container at a fixed position around a rotating wheel. The discharge pipe is fixed at a fixed position such that the discharge pipe faces a mouth portion of the container that moves along an arc-shaped transport path passing directly below the discharge pipe.

Citation List

Patent Literature

[PTL 1] Japanese Unexamined Patent Application Publication No. 2015-157651

Summary of Invention

Technical Problem

The sterilizing unit in PTL 1 continuously discharges the sterilization agent even in a case where the discharge pipe and the mouth portion of the container do not face each other and the phase between the discharge pipe and the mouth portion does not match. Therefore, the sterilization agent discharged while the phase between the discharge pipe and the mouth portion does not match is consumed without being used for sterilization.

As described above, an object of the present disclosure is to provide a supply device that can reduce an amount of a sterilization agent that is not used for sterilization.

Solution to Problem

A sterilization agent supply device according to the present disclosure includes:

a supply unit including a discharge pipe that supplies a sterilization agent downward in a vertical direction at a fixed position; and

a transport unit including a transport body that moves along a transport path while holding a plurality of sterilization target objects by holding tools, and a moving nozzle that is provided on the transport body corresponding to each of the plurality of sterilization target objects and that moves in synchronization with the corresponding sterilization target object.

The moving nozzle includes a first flow passage that receives the sterilization agent upstream and that allows the sterilization agent to flow toward the sterilization target object downstream, and

in the first flow passage, an opening area on an upstream side is larger than an opening area on a downstream side.

A nozzle according to the present disclosure is provided between a discharge pipe and a sterilization target object, and allows a sterilization agent supplied from the discharge pipe to flow toward the sterilization target object.

The nozzle includes a first flow passage that receives the sterilization agent upstream and that allows the sterilization agent to flow toward the sterilization target object downstream, and

in the first flow passage, an opening area on an upstream side is larger than an opening area on a downstream side.

The present disclosure provides a method of sterilizing a sterilization target object by supplying a mist-like sterilization agent discharged from a discharge pipe to the sterilization target object through a nozzle.

The nozzle used in the sterilization method includes a first flow passage that receives the sterilization agent upstream and that allows the sterilization agent to flow toward the sterilization target object downstream.

In the first flow passage, an opening area on an upstream side is larger than an opening area on a downstream side.

Advantageous Effects of Invention

According to the moving nozzle of the supply device according to the present disclosure, in the first flow passage that allows the sterilization agent to flow toward the sterilization target object, the opening area on the upstream side is larger than the opening area on the downstream side. Therefore, since a time during which the phase between a first flow passage discharge pipe and the first flow passage matches on the upstream side can be increased, an amount of the sterilization agent that is not used for sterilization can be reduced.

Brief Description of Drawings

FIG. 1 is a plan view showing a schematic configuration of a supply device according to an embodiment.

FIG. 2 is a side view showing a schematic configuration of a supply device according to a first embodiment.

FIG. 3 is a front sectional view (PSD) and a side sectional view (SSD) showing main parts of the supply device according to the first embodiment.

FIG. 4 is a view showing main parts of the supply device according to the first embodiment and showing a state of movement of one moving nozzle with respect to one fixed nozzle.

FIG. 5 is a view showing main parts of the supply device according to the first embodiment and showing a state of movement of a plurality of moving nozzles with respect to one fixed nozzle.

FIG. 6 is a partial sectional view and a plan view showing a moving nozzle according to the first embodiment.

FIG. 7 is a side sectional view showing the moving nozzle according to the first embodiment.

FIG. 8 is a plan view and a partially enlarged view of the moving nozzle according to the first embodiment.

FIG. 9 is a plan view showing the moving nozzle according to the first embodiment.

FIG. 10 is a front sectional view (PSD) and a side sectional view (SSD) showing a moving nozzle according to a second embodiment.

FIG. 11 is a view showing the moving nozzle according to the second embodiment and showing a state of movement of one moving nozzle with respect to one fixed nozzle.

FIG. 12 is a view showing the moving nozzle according to the second embodiment and showing a state of movement of a plurality of moving nozzles with respect to one fixed nozzle.

FIG. 13 is a view showing a modification example of the moving nozzle according to the second embodiment.

FIG. 14 is a view showing a moving nozzle according to a modification example of the second embodiment and showing a state of movement of one moving nozzle with respect to one fixed nozzle.

FIG. 15 is a view showing another modification example of the moving nozzle according to the second embodiment.

FIG. 16 is a view showing a modification example of a guide vane.

Description of Embodiments

Hereinafter, embodiments will be described with reference to the accompanying drawings. The embodiments described below include a first embodiment and a second embodiment, and in any of the supply devices, a mist-like sterilization agent is supplied to an inside of each of a plurality of containers PB by spraying while the plurality of containers PB move along an arc-shaped transport path. In addition, in any of the supply devices, by including a plurality of moving nozzles 35 that move in synchronization with a holding tool GR that holds the container PB, an amount of the sterilization agent that is not used for sterilization can be reduced. Hereinafter, the first embodiment and the second embodiment will be described in order.

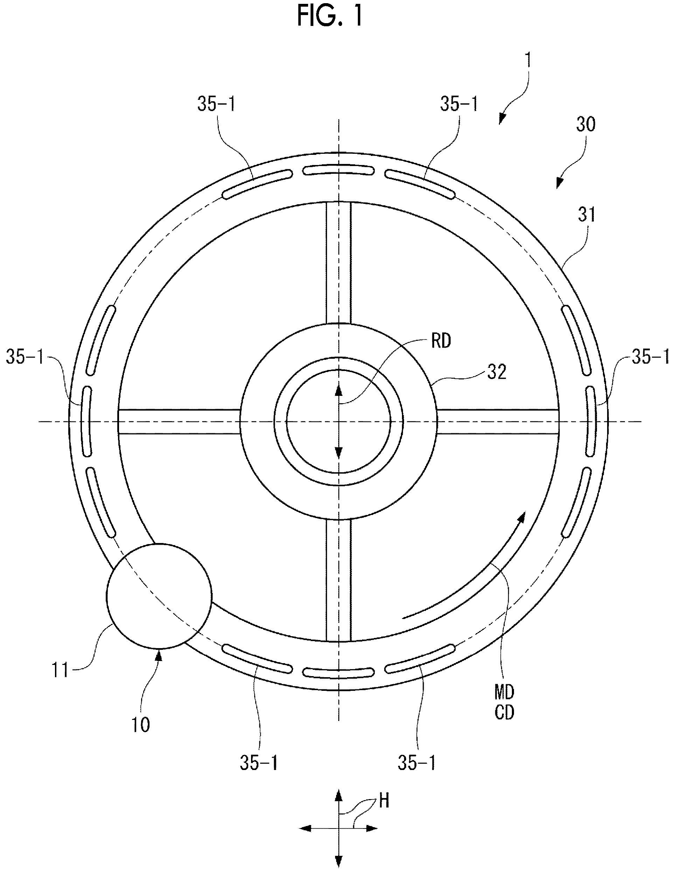

[First Embodiment: Refer to FIGS. 1 to 5]

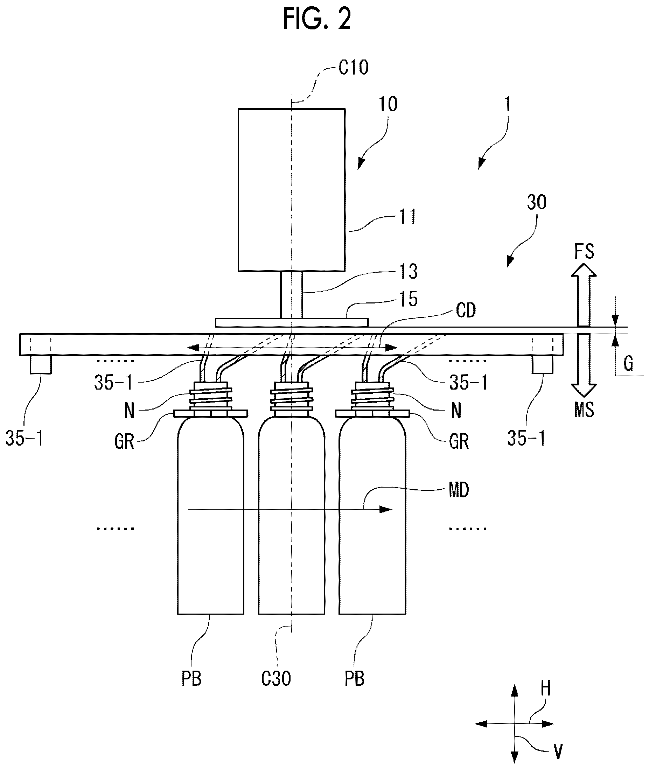

As shown in FIGS. 1 and 2, a supply device 1 according to the first embodiment includes a supply unit 10 that discharges a sterilization agent at a fixed position and supplies the sterilization agent to the container PB, and a transport unit 30 that transports the plurality of containers PB while gripping the plurality of containers PB along the arc-shaped transport path. The supply unit 10 includes, for example, one discharge pipe 13, and the transport unit 30 includes the number of moving nozzles 35-1 corresponding to the number of containers PB to be transported. A supply device 1 according to the second embodiment also has the same configuration except for a structure of the moving nozzle 35-1.

[Supply Unit 10: Refer to FIGS. 1 to 3]

The supply unit 10 includes a mist generator 11 that generates a mist M, which is a mist-like sterilization agent, a discharge pipe 13 that discharges the mist M generated by the mist generator 11 toward the container PB through the moving nozzle 35-1, and a restriction body 15 that suppresses the mist M discharged from the discharge pipe 13 from leaking out from the moving nozzle 35-1. Positions of the mist generator 11, the discharge pipe 13, and the restriction body 15 are fixed.

[Mist Generator 11: Refer to FIGS. 1 and 2]

For example, the mist generator 11 can obtain the mist M by converting a water solution of hydrogen peroxide, which is a sterilization agent, into a droplet shape, and heating the droplet-shaped hydrogen peroxide to a temperature equal to or higher than a boiling point and equal to or lower than a non-decomposition temperature to vaporize the hydrogen peroxide. The mist M is sprayed from the discharge pipe 13.

The droplet-shaped hydrogen peroxide can be obtained, for example, by introducing each of the water solution of hydrogen peroxide and compressed air into a two-fluid spray. In order to vaporize the droplet-shaped hydrogen peroxide, a heater consisting of an electric heating wire or the like may be provided around a pipe line through which the droplet-shaped hydrogen peroxide flows, and the droplet-shaped hydrogen peroxide blown into the pipe line may be heated to a desired temperature. The mist M in which the hydrogen peroxide is vaporized is guided to the discharge pipe 13.

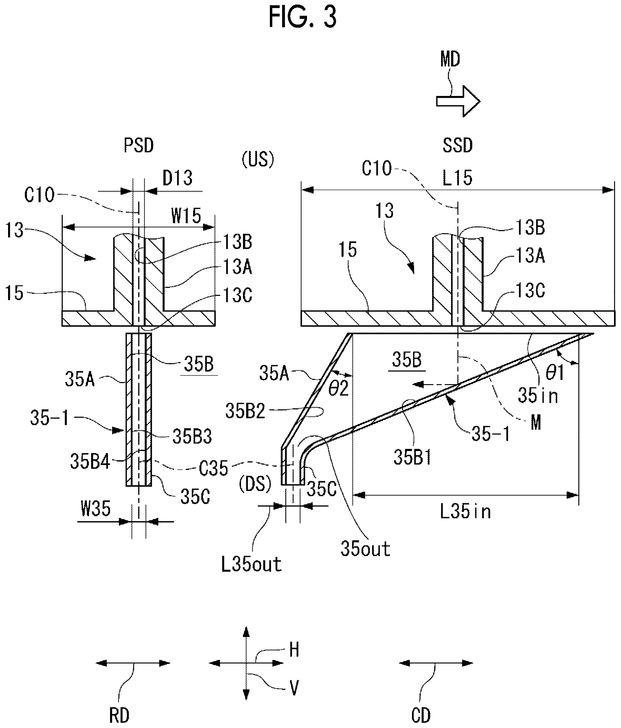

[Discharge Pipe 13: Refer to FIG. 3]

The discharge pipe 13 allows the mist M generated by the mist generator 11 to flow downward in a vertical direction V and discharges the mist M toward the transport unit 30.

The discharge pipe 13 includes, for example, a pipe body 13A made of metal, a passage 13B through which the mist M passes, which is provided inside the pipe body 13A, and a discharge port 13C that is connected to the passage 13B and from which the mist M is discharged.

The discharged mist M is sprayed as a mist toward an opening of a mouth portion N of a bottle PB.

The mist M generated by the mist generator 11 is discharged toward the transport unit 30 through the passage 13B and the discharge port 13C. The passage 13B has, for example, a circular shape according to a shape of the opening of the container PB, and an opening diameter D13 of the passage 13B has a preferable relationship with an opening dimension W35 in a radial direction RD of the moving nozzle 35-1 described below, but the relationship will be described after the moving nozzle 35-1 is described.

[Restriction Body 15: Refer to FIGS. 2 and 3]

As a preferred form, the supply unit 10 includes the restriction body 15 at a lower end of the discharge pipe 13.

The restriction body 15 is provided to suppress the mist M discharged from the discharge port 13C of the discharge pipe 13 from leaking out from the first flow passage 35B of the moving nozzle 35-1. In order to achieve the purpose, the restriction body 15 has a flat portion that closes an opening of the first flow passage 35B on an upstream US side, and has a form in which a shape in a plan view is rectangular as an example, and is provided at the lower end of the discharge pipe 13 to be orthogonal to an axis C10 of the discharge pipe 13 (supply unit 10). A portion of the restriction body 15 corresponding to the discharge port 13C is penetrated in a direction of the axis C10. The restriction body 15 is not limited to the form shown in the drawing. The restriction body 15 shown in the drawing has a flat shape in a side view, but for example, other shapes such as a shape in which both ends in a circumferential direction CD are curved upward as in an arc can be adopted. In addition, the shape in a plan view is not limited to a rectangle, and a shape such as an ellipse or a polygon other than a rectangle can be adopted.

The restriction body 15 has a lowest position in the vertical direction V among members constituting the supply unit 10 disclosed in the embodiment, but a gap G is provided between the restriction body 15 and the transport unit 30. Therefore, the supply unit 10 having a fixed position and the transport unit 30 that rotates do not come into contact with each other during the operation of the supply device 1.

In order to achieve the purpose, several dimensions of the restriction body 15 are specified as described below.

[Transport Unit 30; Refer to FIGS. 1, 2, and 3]

Next, the transport unit 30 will be described.

The transport unit 30 receives and holds the plurality of containers PB continuously carried in from an upstream step in order with the holding tool GR corresponding to each of the plurality of containers PB, and rotates the plurality of containers PB to carry out the plurality of containers PB toward a downstream step. During the period from the carrying-in to the carrying-out, the mist M is supplied to the inside of the container PB, and the sterilization is executed.

The transport unit 30 includes a rotary table 31 and a rotating electric machine 32 that rotationally drives the rotary table 31.

The rotary table 31 has a circular shape in a plan view. A plurality of holding tools GR that grip the mouth portion N of the container PB, which are gripping tools typically referred to as grippers, are provided on an outer peripheral side of the rotary table 31. The plurality of containers PB move along the arc-shaped transport path MD in a state of being held by the holding tools GR, respectively, in association with the rotational drive of the rotary table 31 by the rotating electric machine 32.

A plurality of moving nozzles 35-1 are provided on the rotary table 31. The plurality of moving nozzles 35-1 are arranged at intervals along a circumferential direction CD on an outer peripheral edge of the rotary table 31. The plurality of moving nozzles 35-1 are provided at the same position in the radial direction RD. The plurality of moving nozzles 35-1 are provided corresponding to each of the plurality of holding tools GR, which are not shown in FIGS. 1 and 2. That is, each of the containers PB gripped by the holding tool GR corresponds to the moving nozzle 35-1, and the mist M is supplied to the container PB through the corresponding moving nozzle 35-1. The corresponding holding tool GR and the moving nozzle 35-1 move in synchronization with each other while maintaining a relative positional relationship in association with the rotation of the rotary table 31. Each of the moving nozzles 35-1 is attachably and detachably attached to the rotary table 31 with respect to the rotary table 31. As shown in FIGS. 1 and 2, for example, an upper portion of each of the moving nozzles 35-1 is held on the rotary table 31 by appropriate means, and a lower portion of each of the moving nozzles 35-1 protrudes downward from the rotary table 31. In FIGS. 1 and 2, only a part of the plurality of moving nozzles 35-1 is shown.

As shown in FIG. 3, the moving nozzle 35-1 includes a nozzle housing 35A that constitutes an outer periphery of the moving nozzle 35-1, a first flow passage 35B that is provided inside the nozzle housing 35A, and a discharge port 35C that is connected to the first flow passage 35B. In the moving nozzle 35-1, since the supplied mist M flows from an upper side to a lower side in the drawing, the upstream (US) side and the downstream (DS) side are defined according to the flow. In addition, the moving nozzle 35-1 moves in the circumferential direction CD in association with the rotation of the rotary table 31 together with the container PB, but the position in the radial direction RD is fixed. A movement path MD of the container PB is formed along the circumferential direction CD. In the present embodiment, the moving nozzle 35-1 moves along the movement path MD in a counterclockwise direction in accordance with the direction in which the rotary table 31 rotates. The moving nozzle 35-1 moves from a rear side to a front side in a direction on the movement path MD.

In the moving nozzle 35-1, as shown in FIG. 3, a dimension of the first flow passage 35B in the circumferential direction CD is larger than a dimension in the radial direction RD.

In the first flow passage 35B, an opening dimension L35in of an inlet port 35in on an upstream (US) side is set to be larger than an opening dimension L35out of an outlet port 35out on a downstream (DS) side in the circumferential direction CD. By setting the inlet port 35in on the upstream (US) side to be larger in this way, the distance or time during which the discharge port 13C of the discharge pipe 13 passes above the first flow passage 35B in a process in which the moving nozzle 35-1 moves in synchronization with the container PB can be increased. Therefore, even in a case where the mist M is continuously discharged from the discharge port 13C, an amount of the mist M that is not used for the sterilization of the container PB can be significantly reduced. On the other hand, since the mist M needs to be supplied to the opening of the container PB on the downstream (DS) side, the opening dimension L35out of the outlet port 35out is smaller than the opening dimension L35in, and is particularly preferably set to be equal to or less than a diameter of the opening of the container PB.

Since the dimension W35 of the first flow passage 35B in the radial direction RD is constant, the opening dimension L35in being larger than the opening dimension L35out is equivalent to an opening area of the inlet port 35in on the upstream (US) side being larger than an opening area of the outlet port 35out on the downstream (DS) side.

The flow passage 35B is formed by a pair of circumferential wall surfaces 35B1 and 35B2 and a pair of radial wall surfaces 35B3 and 35B4, excluding the inlet port 35in and the outlet port 35out. The first flow passage 35B is partitioned by the circumferential wall surface 35B1 and the circumferential wall surface 35B2 in the circumferential direction CD, and is partitioned by the radial wall surface 35B3 and the radial wall surface 35B4 in the radial direction RD.

In the movement direction of the moving nozzle 35-1, the circumferential wall surface 35B1 is positioned in front of the circumferential wall surface 35B2. Whether or not the circumferential wall surface 35B1 is positioned in front of the circumferential wall surface 35B2 is determined by observing the entire circumferential wall surface 35B1 and the entire circumferential wall surface 35B2. In addition, the circumferential wall surface 35B1 corresponds to a first circumferential wall surface in the present disclosure, and the circumferential wall surface 35B2 corresponds to a second circumferential wall surface in the present disclosure.

The circumferential wall surfaces 35B1 and 35B2 satisfy the following three requirements. The moving nozzle 35-1 can exhibit an action and an effect described below in the supply of the mist M to the container PB by satisfying the following three requirements. The inclinations ($\theta1$, $\theta2$) of the circumferential wall surfaces 35B1 and 35B2 refer to inclinations with respect to the vertical direction V in a state where the moving nozzle 35-1 is mounted on the supply device 1.

Requirement 1: In the moving nozzle 35-1, the circumferential wall surface 35B1 and the circumferential wall surface 35B2 are inclined in the same direction in the circumferential direction CD.

Requirement 2: The circumferential wall surface 35B1 and the circumferential wall surface 35B2 are inclined forward in a direction in which the moving nozzle 35-1 moves.

Requirement 3: In a case where the inclinations of the circumferential wall surface 35B1 and the circumferential wall surface 35B2 with respect to the vertical direction V are denoted by $\theta1$ and $\theta2$, respectively, $\theta1 > \theta2$, that is, the inclination of the circumferential wall surface 35B2 positioned behind in the movement direction of the moving nozzle 35-1 is smaller than the inclination of the circumferential wall surface 35B1.

Since the radial wall surfaces 35B3 and 35B4 are provided with the circumferential wall surfaces 35B1 and 35B2, the radial wall surfaces 35B3 and 35B4 continuously decrease from an inlet port 35in side to an outlet port 35out side. That is, the volume of the first flow passage 35B also continuously decreases from the inlet port 35in side to the outlet port 35out side. However, for example, the opening dimension of the first flow passage 35B in the circumferential direction (CD) may be formed to intermittently decrease from the upstream US side to the downstream DS side.

The discharge port 35C is connected to the outlet port 35out of the first flow passage 35B and has the same opening dimension as the outlet port 35out.

Specific dimensions in the horizontal direction H and the vertical direction V are optional, and may be appropriately set according to specifications or the like of the supply unit 10 and the transport unit 30.

[Dimensional Relationship between Discharge Pipe 13, Restriction Body 15, and Moving Nozzle 35-1: Refer to FIG. 3]

First, a dimensional relationship between the discharge pipe 13 and the moving nozzle 35-1 will be described.

A diameter (opening dimension) D13 of the discharge port 13C of the discharge pipe 13 is preferably smaller than the opening dimension W35 of the first flow passage 35B of the moving nozzle 35-1 in the radial direction RD. This is to reduce the amount of the mist M discharged and sprayed from the discharge port 13C that leaks to the periphery without entering the first flow passage 35B. On the contrary, in a case where the diameter D13 is larger than the opening dimension W35, the amount of the mist M discharged from the discharge port 13C that leaks out in the radial direction RD without entering the first flow passage 35B increases. An opening dimension L35in of the first flow passage 35B on the inlet port 35in side is significantly larger than the diameter D13. The above-described relationship is summarized below.

$$D13 < W35,\ D13 \ll L35in$$

Next, a dimensional relationship between the restriction body 15 and the moving nozzle 35-1 will be described.

A dimension L15 of the restriction body 15 in the circumferential direction CD is preferably larger than the opening dimension L35in of the first flow passage 35B of the moving nozzle 35-1 on the inlet port 35in side. In addition, a dimension W15 of the restriction body 15 in the radial direction RD is preferably larger than the opening dimension W35 of the first flow passage 35B of the moving nozzle 35-1 on the inlet port 35in side. As a result, the mist M temporarily supplied to the first flow passage 35B can be suppressed from leaking out from the first flow passage 35B.

$$L15 > L35in,\ W15 > W35,\ L15 > W15$$

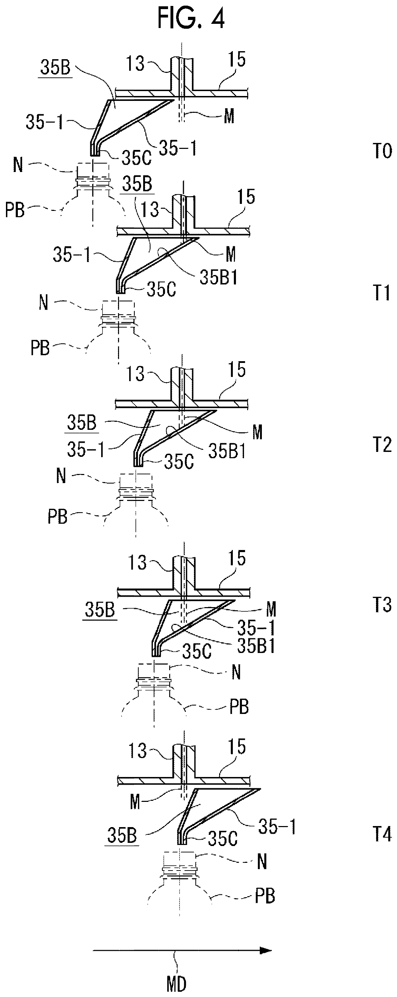

[Basic Operation of Moving Nozzle 35-1: Refer to FIG. 4]

Next, a basic operation of the moving nozzle 35-1 with respect to the discharge pipe 13 in a case of sterilizing the container PB by the supply device 1 will be described with reference to only one moving nozzle 35-1. In FIG. 4, the time series is from the top to the bottom. That is, in FIG. 4, the moving nozzle 35-1 moves along the transport path MD in the circumferential direction CD in the order of T0, T1, T2, T3, and T4.

The moving nozzle 35-1 moves in synchronization with the movement of the container PB with respect to the discharge pipe 13 having a fixed position (T0), but at this point in time, the flow passage 35B of the moving nozzle 35-1 has not reached the discharge port 13C of the discharge pipe 13. Therefore, the mist M discharged from the discharge port 13C is not supplied to the flow passage 35B of the moving nozzle 35-1.

The moving nozzle 35-1 continues to move, and the first flow passage 35B reaches directly below the discharge pipe 13 (T1). Therefore, the mist M is discharged from the discharge port 13C toward the first flow passage 35B, and is supplied to the inside of the container PB through the first flow passage 35B, the discharge port 35C, and the mouth portion N.

Further, even in a case where the moving nozzle 35-1 continues to move (T2, T3), the first flow passage 35B is directly below the discharge port 13C, and the first flow passage 35B is related to the discharge pipe 13. Therefore, the mist M is discharged from the discharge port 13C toward the first flow passage 35B, and the sterilization agent is continuously supplied to the inside of the container PB through the first flow passage 35B, the discharge port 35C, and the mouth portion N.

Further, in a case where the moving nozzle 35-1 continues to move, the moving nozzle 35-1 deviates from directly below the discharge pipe 13 and is separated from the discharge pipe 13 (T4), and the supply of the mist M to the container PB ends.

As described above, the mist M received by one moving nozzle 35-1 from the discharge pipe 13 is supplied to the container PB through the moving nozzle 35-1 while the first flow passage 35B passes through the discharge pipe 13. In a case where the mist M is directly supplied from the discharge pipe 13 to the mouth portion N of the container PB without passing through the moving nozzle 35-1, the mist M is supplied to the inside of the container PB only for a short period of time in which the discharge pipe 13 and the container PB overlap. Therefore, in a case where the mist M is continuously discharged from the discharge pipe 13, a large amount of the mist M is not used for the sterilization.

In the supply operation of the mist M, the following action occurs in the supply of the mist M by satisfying the three requirements described above.

Action of Requirement 1 (inclined in the same direction): While the first flow passage 35B passes through the discharge pipe 13 (T1 to T4 in FIG. 4), the mist M always collides only with the circumferential wall surface 35B1, and the mist M after the collision is aligned with the flow toward the circumferential wall surface 35B2 in the circumferential direction CD (FIG. 3). Therefore, the direction of the mist M flowing out from the discharge port 35C is also the same, the jet of the mist M to the container PB is stabilized, and a stable scavenging flow is formed inside the container PB, so that the scavenging efficiency is improved.

Action of Requirement 2 (inclined in the moving direction): Since the relative speed of the mist M colliding with the circumferential wall surface 35B1 can be reduced as compared with a case where the inclination is inclined in a direction opposite to the moving direction of the moving nozzle 35-1, the collision of the mist M with the circumferential wall surface 35B1 can be mitigated. Therefore, the air current fluctuation (vortex flow) during the collision of the mist M with the circumferential wall surface 35B2 is suppressed, and the mixing of the mist M with the surrounding air in the first flow passage 35B is suppressed. As a result, a decrease in concentration of the sterilization agent, the sterilant, for example, hydrogen peroxide ($H_2O_2$) in the mist M discharged from the discharge port 35C can be suppressed.

Action of Requirement 3 ($\theta 1 > \theta 2$): By setting the inclination $\theta 1$ to be larger than the inclination $\theta 2$ to bring the circumferential wall surface 35B1 closer to the horizontal direction (H), the opening dimension L35out of the discharge port 35C can be minimized. As a result, since the flow of the mist M along the circumferential wall surface 35B1 can be smoothly discharged from a discharge hole 35C, the separation of the flow of the mist M and the mixing with the surrounding air due to the vortex flow are suppressed. As a result, the concentration of the mist M discharged from the discharge hole 35C can be maintained. The opening dimension L35out of the discharge port 35C can be minimized by setting the inclination $\theta 1$ to be larger than the inclination $\theta 2$ on the premise that the opening dimension L35in, the dimension of the first flow passage 35B in the vertical direction V, and the inclination $\theta 2$ are the same.

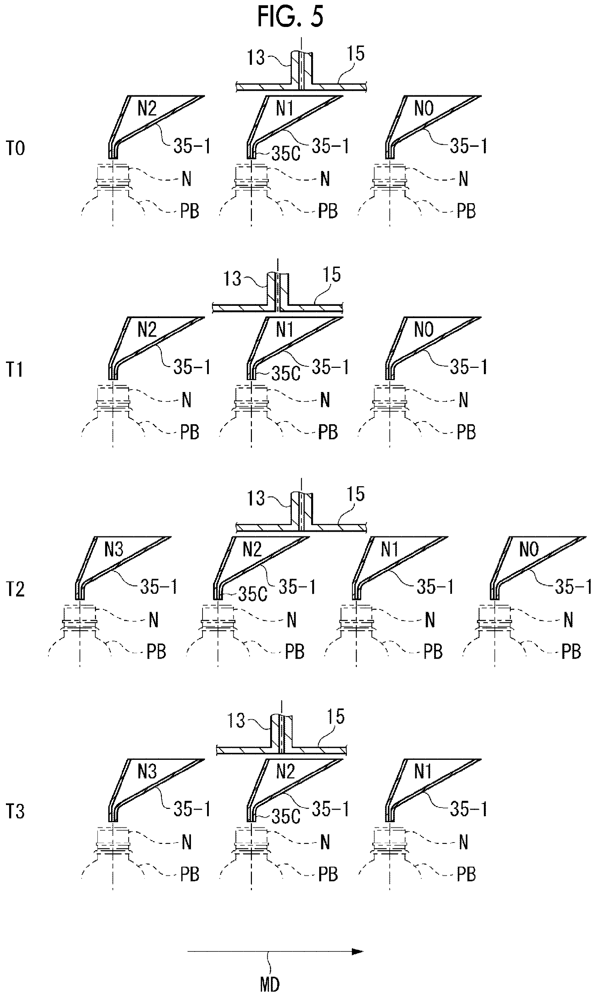

[Operation by Plurality of Moving Nozzles 35-1: Refer to FIG. 5]

In the above, the operation with respect to the discharge pipe 13 has been described by focusing on only one moving nozzle 35-1, but in reality, since a plurality of moving nozzles 35-1 are arranged in the circumferential direction CD, that is, the movement path MD, the moving nozzles 35-1 are involved in the discharge pipe 13 in front and rear. Hereinafter, description will be made with reference to FIG. 5. In FIG. 5, the time series is from the top to the bottom. In addition, in FIG. 5, N0, N1, ..., and the like are symbols for identifying the moving nozzle 35-1, and N0, N1, ..., and the like pass through the discharge pipe 13 in the order of N0, N1, ..., and the like.

For example, it is assumed that the moving nozzle 35-1 (N1) has reached directly below the discharge pipe 13, and the moving nozzle 35-1 (N0) and the moving nozzle 35-1 (N2) are arranged in front and rear of the moving nozzle 35-1 (N1) (T0). In this case, the container PB corresponding to the moving nozzle 35-1 (N1) is in the middle of being supplied with the mist M from the discharge pipe 13, whereas the container PB corresponding to the moving nozzle 35-1 (N0) has already ended the supply of the mist M to the inside. The container PB corresponding to the moving nozzle 35-1 (N2) is in a stage before the supply of the mist M.

In a case where the movement of the moving nozzle 35-1 (N0) proceeds (T1 to T3), the overlap between the moving nozzle 35-1 (N1) and the discharge pipe 13 ends (T2), and the moving nozzle 35-1 (N2) and the discharge pipe 13 overlap (T2, T3), and the supply of the mist M to the inside of the container PB corresponding to the moving nozzle 35-1 (N2) starts.

Thereafter, in the same manner, in a case where the supply of the mist M to the inside of the container PB corresponding to the moving nozzle 35-1 (N2) ends, the operation of starting and ending the supply of the mist M to the inside of the container PB corresponding to the next moving nozzle 35-1 (N3) is repeated.

Here, in a case where the opening dimension of the inlet port 35in of each of the moving nozzles 35-1 in the circumferential direction CD is denoted by L35in, and the dimension of the interval between the inlet ports 35in and 35in of the adjacent moving nozzles 35-1 and 35-1 is denoted by D, L35in > D is established. It is assumed that a total of N moving nozzles 35-1 are provided on the rotary table 31. In this case, in a case where the total length of the circumferential direction CD in which the mist M is supplied to the first flow passage 35B of all the moving nozzles 35-1 while the rotary table 31 rotates once is L35in $\times$ N, the total length of the circumferential direction CD in which the mist M is not supplied to the first flow passage 35B is D $\times$ N, and the relationship of L35in $\times$ N > D $\times$ N is established. That is, according to the present embodiment, the distance or time in which the discharge pipe 13 and the moving nozzle 35-1 are involved is significantly larger, for example, 2 times or more and further 3 times or more, than the distance or time in which the discharge pipe 13 and the interval between the moving nozzles 35-1 and 35-1 are involved. The same applies to a moving nozzle 35-2 described below.

[Effects of First Embodiment]

<First Effect>

According to the moving nozzle 35-1 according to the first embodiment, the mist M can be discharged to the inside of the corresponding container PB while the phase between the discharge pipe 13 and the inlet port 35in of the moving nozzle 35 matches. Since the opening dimension L35in of the inlet port 35in in the circumferential direction CD is several times larger than the opening dimension of the mouth portion N of the container PB, the time during which the phase between the discharge pipe 13 and the inlet port 35in matches is long. As a result, the amount of the mist M that can be supplied to the inside of the container PB from one discharge pipe 13 can be increased. For example, the number of discharge pipes 13 can be reduced, and the amount of the sterilization agent used can be reduced by interposing the moving nozzle 35 as compared with a case where the same amount of the mist M is directly supplied to the inside of the container PB from the discharge pipe 13.

<Second Effect>

The moving nozzle 35-1 can improve the scavenging efficiency, and can suppress a decrease in concentration of the sterilization agent, for example, hydrogen peroxide, supplied to the container PB by satisfying the three requirements described above.

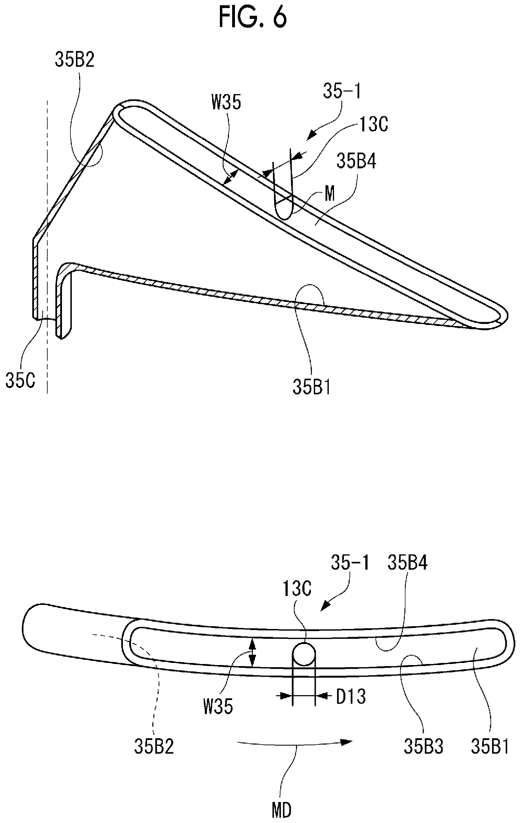

[Preferred Example of First Embodiment: FIGS. 6 to 9]

Hereinafter, some preferred aspects of the moving nozzle 35-1 will be described.

[Relationship between Opening Dimension W35 and Diameter D13: Refer to FIG. 6]

It is preferable that the opening dimension W35 specified by the interval between the radial wall surface 35B3 and the radial wall surface 35B4 of the moving nozzle 35-1 is larger than the diameter D13 of the discharge port 13C. In this manner, since the mist M blown into the moving nozzle 35-1 can flow into the inside without being blocked by the upper surface of the moving nozzle 35-1, the consumption amount of the sterilization agent can be suppressed. Since the mist M spreads and travels from the discharge port 13C, it is more preferable that the opening dimension W35 is larger than the spreading of the mist M.

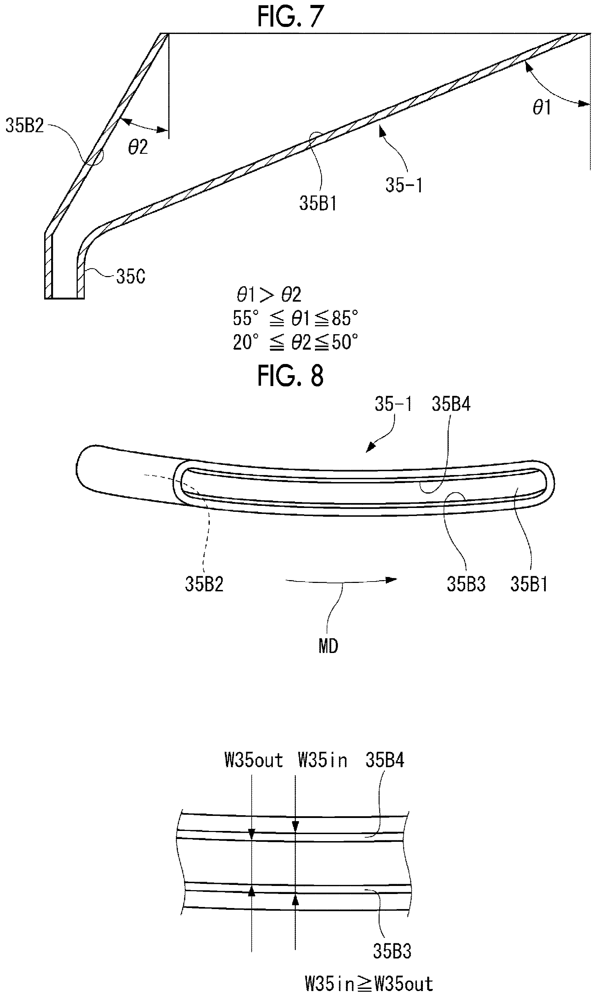

[Inclinations θ2, θ1: Refer to FIG. 7]

Next, in addition to θ1 > θ2 for the inclinations θ1 and θ2 of the circumferential wall surface 35B1 and the circumferential wall surface 35B2, θ2 is preferably 20° or more and more preferably 30° or more from the vertical direction V.

Since the Coanda effect acts between the mist M flowing as a jet and the circumferential wall surface 35B2, in a case where the inclination θ2 is small, the mist M adheres to and remains on the circumferential wall surface 35B2. In this case, since the blowing-out direction of the mist M at the discharge hole 35C changes, the effect of Requirement 1 described above is reduced. The Coanda effect refers to an effect in which a jet is attracted to a wall surface by forming a separation vortex between the jet and the wall surface and reducing static pressure.

On the other hand, by setting θ2 to equal to or more than 20°, since the straightness of the mist M as a jet is superior to the Coanda effect, and the mist M can be caused to collide with the circumferential wall surface 35B1, the effect of Requirement 1 can be reliably obtained.

On the other hand, in a case where θ2 is excessively large, the area of the circumferential wall surface 35B2 that covers the circumferential wall surface 35B1 is large, and the area of the circumferential wall surface 35B1 that receives the mist M is small. Therefore, the preferred θ2 is 50° or less, and the more preferred θ2 is 40° or less.

In addition, in order to receive the mist M for a longer period of time by increasing the dimension of the circumferential wall surface 35B1 in the circumferential direction CD, the preferred θ1 is 55° or more, and the more preferred θ1 is 65° or more. On the other hand, in a case where θ1 is excessively large and the circumferential wall surface 35B1 approaches the horizontal direction H, since it is difficult to cause the mist M received to flow toward the discharge hole 35C, the preferred θ1 is 85° or less, and the more preferred θ1 is 75° or less.

[Reduction of Opening Dimension W35: Refer to FIG. 8]

The opening dimension W35 can be constant from the inlet port 35in to the outlet port 35out, but can be reduced from the inlet port 35in to the outlet port 35out. That is, in a case where the opening dimension at the inlet port 35in is denoted by W35in and the opening dimension at the outlet port 35out is denoted by W35out, it is preferable to satisfy the following. The reduction from the opening dimension W35in to the opening dimension W35out may be continuous or stepwise.

$$W35\text{in} \geq W35\text{out}$$

In a case where the opening dimension W35 satisfies the above-described expression, the flow of the mist M can be suppressed from being disturbed and fluctuating. As a result, the mixing of the mist M with the air in the first flow passage 35B inside the moving nozzle 35-1 is suppressed, and the high-concentration mist M can be blown out from the discharge hole 35C. In addition, since the jet of the mist M can be prevented from decelerating due to the spreading of the flow of the mist M, the time until the mist M reaches the discharge hole 35C can be minimized.

In this case, it is preferable to satisfy the following expression.

$$W35\text{in} \geq D13$$

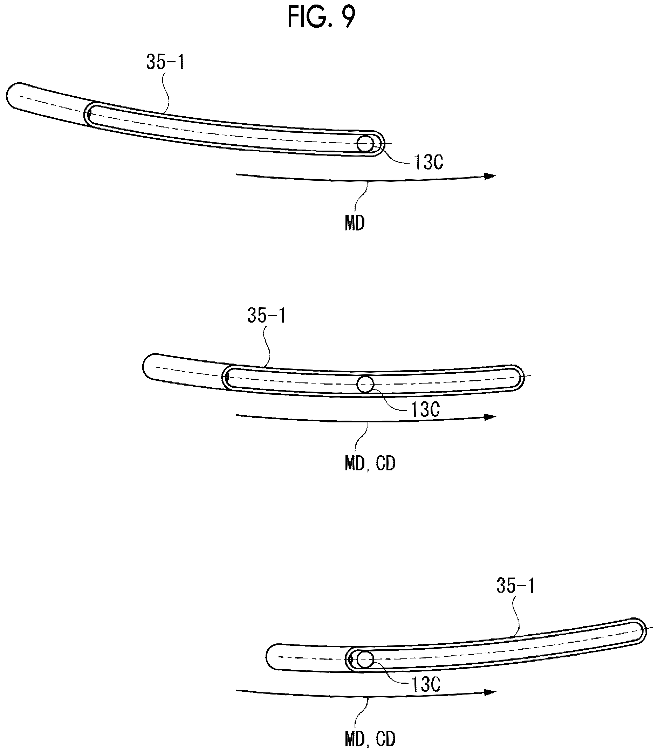

[Curvature of Circumferential Wall Surface 35B1 and Circumferential Wall Surface 35B2: Refer to FIG. 9]

It is preferable that the circumferential wall surface 35B1 and the circumferential wall surface 35B2 are provided with a curvature (k35). By setting the curvature (k35) to be the same as the curvature (k31) of the rotary table 31 (k35 = k31), the discharge port 13C can move to the same position in the radial direction RD of the inlet port 35in of the moving nozzle 35-1.

As a result, even in a case where the moving nozzle 35-1 moves in the circumferential direction CD, since the position of the discharge port 13C in the radial direction RD with respect to the inlet port 35in of the mist M is fixed, the path through which the mist M flows in the first flow passage 35B is stabilized, and the direction in which the mist M flows out from the discharge hole 35C is stabilized. Therefore, since the blowing of the mist M into the container PB disposed close to the discharge hole 35C is stabilized, and a stable scavenging flow is formed inside the container PB, the scavenging efficiency can be improved.

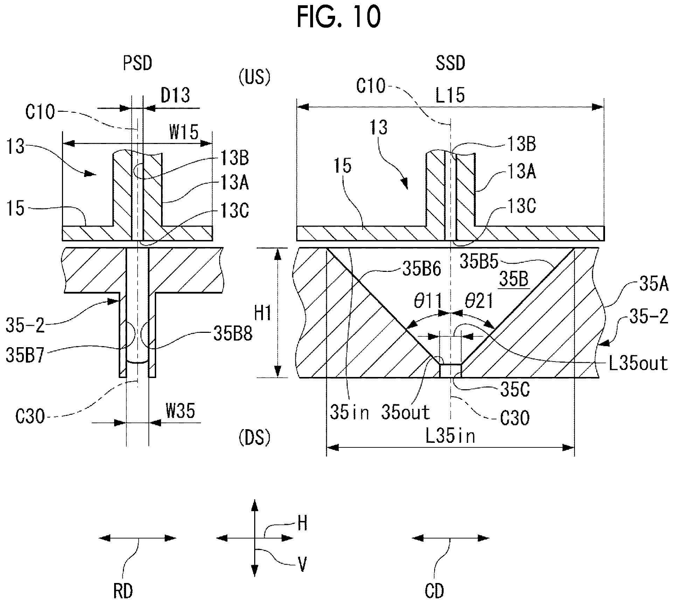

[Second Embodiment: FIGS. 10 to 16]

Next, the moving nozzle 35-2 according to the second embodiment will be described with reference to FIGS. 10 to 16.

The moving nozzle 35-2 according to the second embodiment is the same as the moving nozzle 35-1 according to the first embodiment in that the opening area on the upstream side of the first flow passage 35B is larger than the opening area on the downstream side, but a direction in which the circumferential wall surface 35B6 corresponding to the circumferential wall surface 35B2 is inclined is different. However, the first effect of the moving nozzle 35-1 can be exhibited. Hereinafter, the moving nozzle 35-2 will be described. It should be noted that a portion common to the moving nozzle 35-1 may not be described.

[Structure of Moving Nozzle 35-2: Refer to FIG. 10]

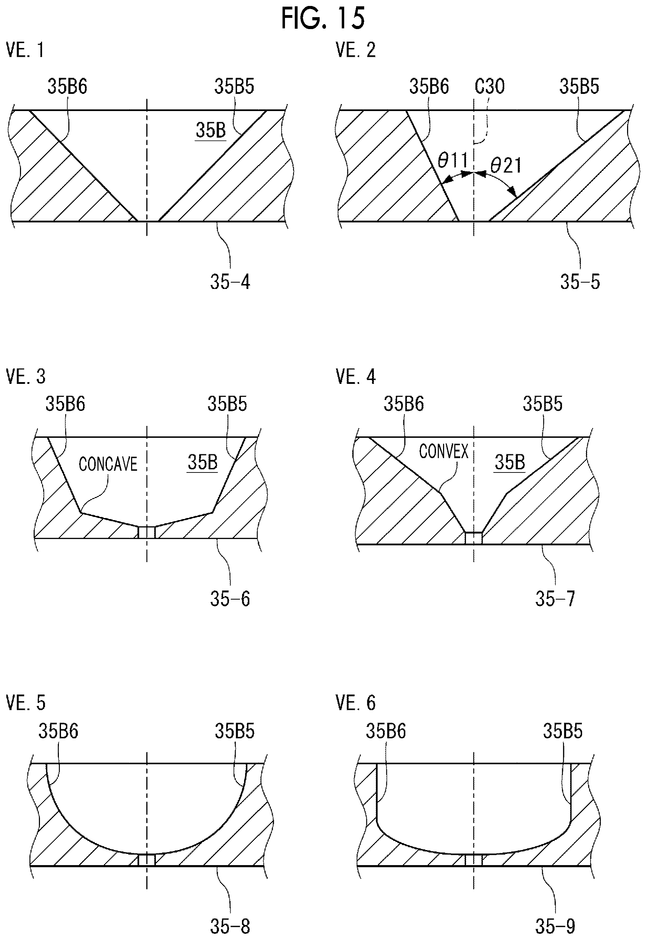

In the moving nozzle 35-2, the circumferential wall surfaces 35B5 and 35B6 are line-symmetric with respect to the central axis C30, and are composed of planes in which angles θ11 and θ21 with the axis C10 match. Therefore, in the circumferential direction CD, the flow passage 35B has a symmetric shape with respect to the central axis C30, the circumferential wall surface 35B5 and the circumferential wall surface 35B6 have a symmetric shape in the circumferential direction CD, and the circumferential wall surface 35B5 and the circumferential wall surface 35B6 are inclined in different directions. However, an example in which the angle θ11 ≠ the angle θ21 is allowed as shown in VE.2 of FIG. 15 described below. An opening dimension W35 of the circumferential wall surfaces 35B5 and 35B6 in a side view is constant and matches an opening dimension L35out of the outlet port 35out on the downstream (DS) side.

Since the radial wall surfaces 35B7 and 35B8 are provided with the circumferential wall surfaces 35B5 and 35B6, the radial wall surfaces 35B7 and 35B8 continuously decrease from the inlet port 35in side to the outlet port 35out side. That is, the volume of the flow passage 35B also continuously decreases from the inlet port 35in side to the outlet port 35out side.

[Dimensional Relationship between Discharge Pipe 13, Restriction Body 15, and Moving Nozzle 35-2: Refer to FIG. 10]

First, a dimensional relationship between the discharge pipe 13 and the moving nozzle 35-2 will be described.

A diameter (opening dimension) D13 of the discharge port 13C of the discharge pipe 13 is preferably smaller than the opening dimension W35 of the flow passage 35B of the moving nozzle 35-2 in the radial direction RD. This is to reduce the amount of the mist M discharged and sprayed from the discharge port 13C that leaks to the periphery without entering the flow passage 35B. On the contrary, in a case where the diameter D13 is larger than the opening dimension W35, the amount of the mist M discharged from the discharge port 13C that leaks out in the radial direction RD without entering the flow passage 35B increases. An opening dimension L35in of the flow passage 35B on the inlet port 35in side is significantly larger than the diameter D13. The above-described relationship is summarized below.

$$D13 < W35,\ D13 \ll L35in$$

Next, a dimensional relationship between the restriction body 15 and the moving nozzle 35-2 will be described.

A dimension L15 of the restriction body 15 in the circumferential direction CD is preferably larger than the opening dimension L35in of the flow passage 35B of the moving nozzle 35-2 on the inlet port 35in side. In addition, a dimension W15 of the restriction body 15 in the radial direction RD is preferably larger than the opening dimension W35 of the flow passage 35B of the moving nozzle 35-2 on the inlet port 35in side. As a result, the mist M temporarily supplied to the flow passage 35B can be suppressed from leaking out from the flow passage 35B.

$$L15 > L35in,\ W15 > W35,\ L15 > W15$$

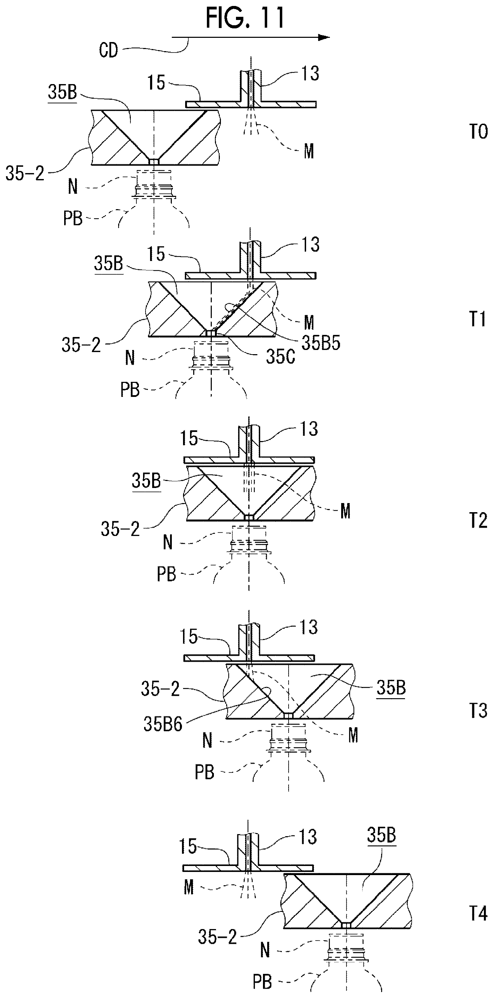

[Basic Operation of Moving Nozzle 35-2: Refer to FIG. 11]

Next, a basic operation of the moving nozzle 35-2 with respect to the discharge pipe 13 in a case of sterilizing the container PB by the supply device 1 will be described with reference to only one moving nozzle 35-2. In FIG. 11, the time series is from the top to the bottom. That is, in FIG. 11, the moving nozzle 35-2 moves along the transport path MD in the circumferential direction CD in the order of T0, T1, T2, T3, and T4.

The moving nozzle 35-2 moves in synchronization with the movement of the container PB with respect to the discharge pipe 13 having a fixed position (T0), but at this point in time, the flow passage 35B of the moving nozzle 35-2 has not reached the discharge port 13C of the discharge pipe 13. Therefore, the mist M discharged from the discharge port 13C is not supplied to the flow passage 35B of the moving nozzle 35-2.

The moving nozzle 35-2 continues to move, and the flow passage 35B reaches directly below the discharge pipe 13 (T1). Therefore, the mist M is discharged from the discharge port 13C toward the flow passage 35B, and is supplied to the inside of the container PB through the flow passage 35B, the discharge port 35C, and the mouth portion N.

Further, even in a case where the moving nozzle 35-2 continues to move (T2, T3), the flow passage 35B is directly below the discharge port 13C, and the flow passage 35B is related to the discharge pipe 13. Therefore, the mist M is discharged from the discharge port 13C toward the flow passage 35B, and the sterilization agent is continuously supplied to the inside of the container PB through the flow passage 35B, the discharge port 35C, and the mouth portion N. The circumferential wall surface 35B5 receives the mist M in the moving nozzle 35-2 in the first half (T1 to T2), but the circumferential wall surface 35B6 receives the mist M in the second half (T2 to T3). The mist M that collides with the circumferential wall surface 35B5 in the first half changes the flow toward the rear side in the movement direction of the moving nozzle 35-2, and the mist M that collides with the circumferential wall surface 35B6 in the second half changes the flow toward the front side in the movement direction of the moving nozzle 35-2.

Further, in a case where the moving nozzle 35-2 continues to move, the moving nozzle 35-2 deviates from directly below the discharge pipe 13 and is separated from the discharge pipe 13 (T4), and the supply of the mist M to the container PB ends.

As described above, the mist M received by one moving nozzle 35-2 from the discharge pipe 13 is supplied to the container PB through the moving nozzle 35-2 while the flow passage 35B passes through the discharge pipe 13 in the circumferential direction CD. In a case where the mist M is directly supplied from the discharge pipe 13 to the mouth portion N of the container PB without passing through the moving nozzle 35-2, the mist M is supplied to the inside of the container PB only for a short period of time in which the discharge pipe 13 and the container PB overlap. Therefore, in a case where the mist M is continuously discharged from the discharge pipe 13, a large amount of the mist M is not used for the sterilization.

As can be understood from FIG. 11, the circumferential wall surface 35B5 receives the mist M in the moving nozzle 35-2 in the first half, but the circumferential wall surface 35B6 receives the mist M in the second half.

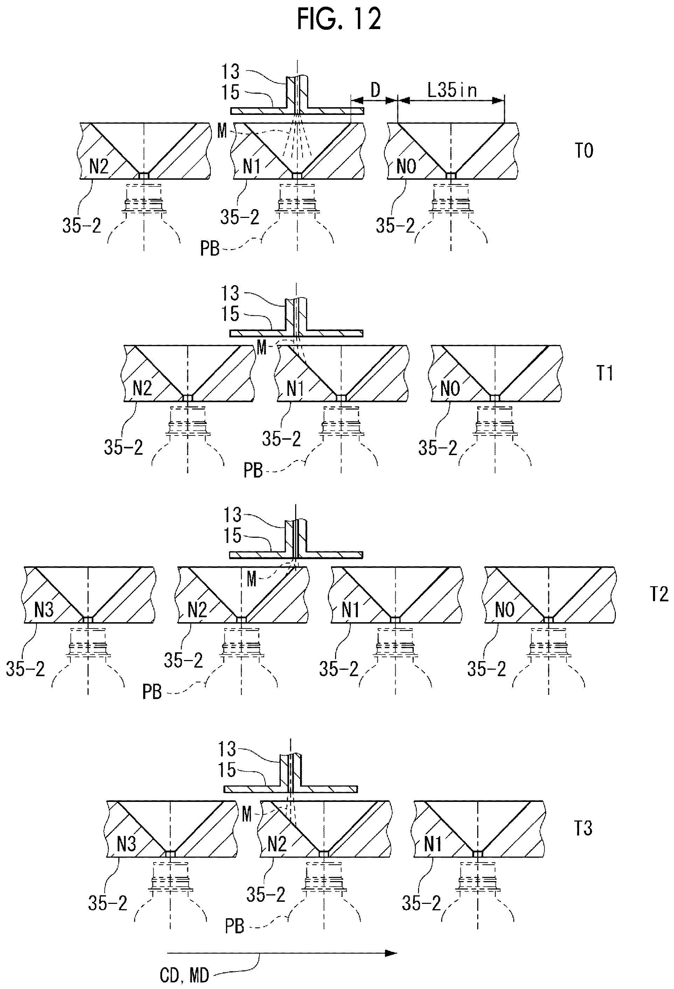

[Operation by Plurality of Moving Nozzles 35-2: Refer to FIG. 12]

In the above, the operation with respect to the discharge pipe 13 has been described by focusing on only one moving nozzle 35-2, but in reality, since a plurality of moving nozzles 35-2 are arranged in the circumferential direction CD, that is, the transport path MD, the moving nozzles 35-2 are involved in the discharge pipe 13 in front and rear. Hereinafter, description will be made with reference to FIG. 12. In FIG. 12, the time series is from the top to the bottom. In addition, in FIG. 12, N0, N1, ..., and the like are symbols for identifying the moving nozzle 35-2, and N0, N1, ..., and the like pass through the discharge pipe 13 in the order of N0, N1, ..., and the like.

For example, it is assumed that the moving nozzle 35-2 (N1) has reached directly below the discharge pipe 13, and the moving nozzle 35-2 (N0) and the moving nozzle 35-2 (N2) are arranged in front and rear of the moving nozzle 35-2 (N1) (T0). In this case, the container PB corresponding to the moving nozzle 35-2 (N1) is in the middle of being supplied with the mist M from the discharge pipe 13, whereas the container PB corresponding to the moving nozzle 35-2 (N0) has already ended the supply of the mist M to the inside. The container PB corresponding to the moving nozzle 35-2 (N2) is in a stage before the supply of the mist M.

In a case where the movement of the moving nozzle 35-2 (N0) proceeds (T1 to T3), the overlap between the moving nozzle 35-2 (N1) and the discharge pipe 13 ends (T2), and the moving nozzle 35-2 (N2) and the discharge pipe 13 overlap (T2, T3), and the supply of the mist M to the inside of the container PB corresponding to the moving nozzle 35-2 (N2) starts.

Thereafter, in the same manner, in a case where the supply of the mist M to the inside of the container PB corresponding to the moving nozzle 35-2 (N2) ends, the operation of starting and ending the supply of the mist M to the inside of the container PB corresponding to the next moving nozzle 35-2 (N3) is repeated.

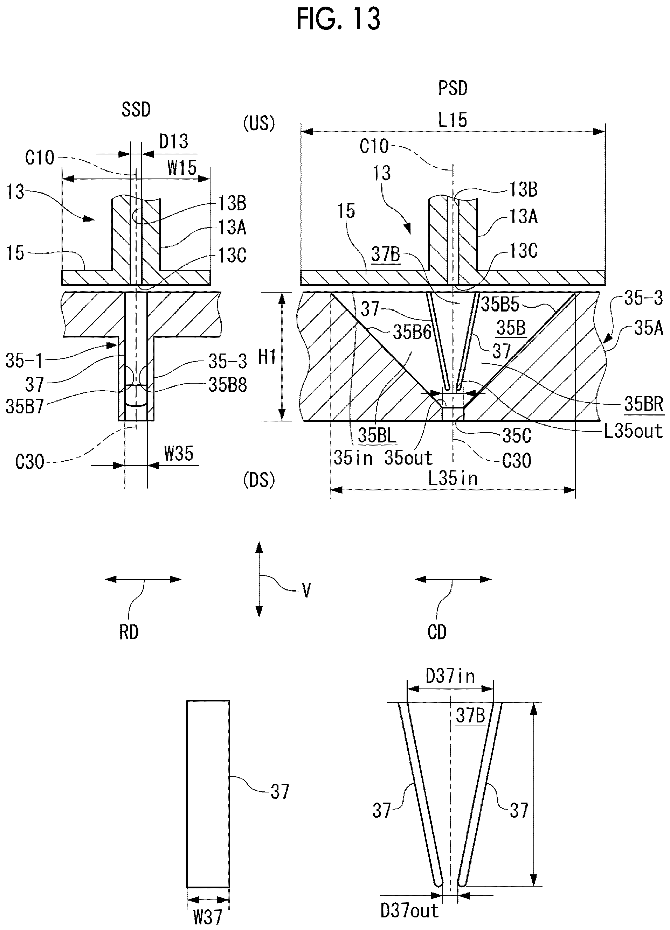

[Configuration of Guide Vane 37: Refer to FIG. 13]

Next, a moving nozzle 35-3 including a pair of guide vanes 37 in the first flow passage 35B will be described. The moving nozzle 35-3 has the same configuration as the moving nozzle 35-2, except for the guide vane 37. The pair of guide vanes 37 are provided for the following purpose.

The pair of guide vanes 37 and 37 suppress a decrease in the amount of the mist M as the sterilization agent supplied to the container PB.

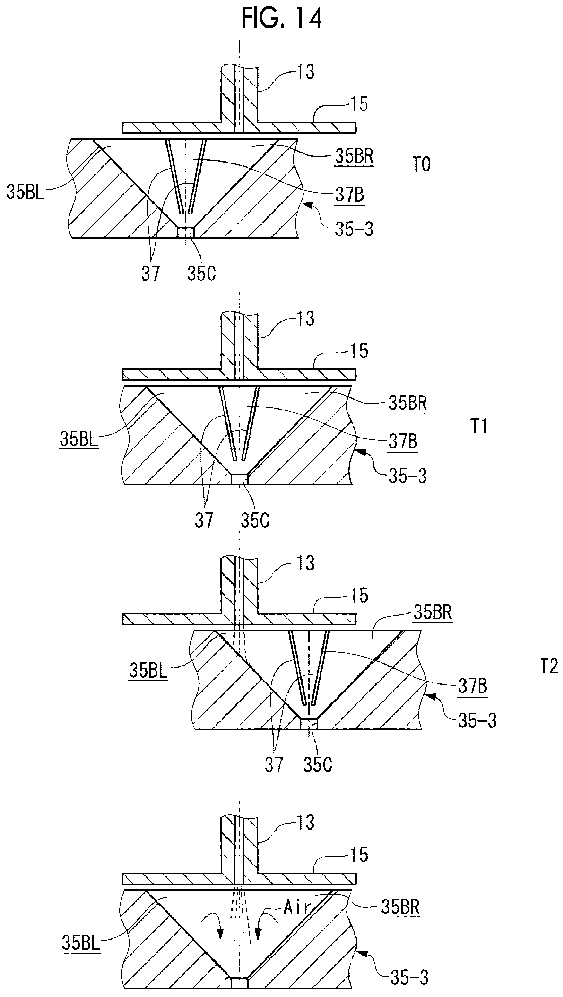

The mist M discharged from the discharge pipe 13 forms a jet in the first flow passage 35B, but the jet entrains the surrounding air Air inside the first flow passage 35B (lowermost side in FIG. 14). The mist M has a temperature of, for example, 150°C or higher, whereas the temperature of the surrounding air is significantly lower than the temperature of the mist M. Therefore, since the temperature of the mist M that entrains the air is lowered, a part of the mist M is condensed, and the mist M remains inside the moving nozzle 35-2 without being supplied to the container PB, the amount of the mist M supplied to the inside of the container PB is reduced. Therefore, in the moving nozzle 35-3, the pair of guide vanes 37 and 37 are provided to suppress the entrainment of the air into the mist M.

The moving nozzle 35-3 includes the pair of guide vanes 37 and 37 inside the first flow passage 35B, and the guide vanes 37 and 37 are arranged in a tapered shape in which an interval D37out on the outlet port 35out side is narrower than an interval D37in on the upstream (US) side in the circumferential direction CD.

That is, as a preferred form, the guide vanes 37 and 37 are tapered in the vertical direction V in the second flow passage 37B inside the guide vanes 37 and 37. The pair of guide vanes 37 and 37 are connected to the entire region in the radial direction RD in the first flow passage 35B. That is, a dimension W37 of the guide vane 37 in the radial direction RD matches the opening dimension W35 of the moving nozzle 35-3 in the radial direction RD. In this way, the second flow passage 37B is formed in a region inside the first flow passage 35B and surrounded by the pair of guide vanes 37 and 37 and the pair of radial wall surfaces 35B7 and 35B8. A left side in the drawing across the second flow passage 37B is referred to as a first-2 flow passage 35BL, and a right side in the drawing is referred to as a first-1 flow passage 35BR. The mist M discharged to the moving nozzle 35-3 flows in the order of the first-2 flow passage 35BL, the second flow passage 37B, and the first-1 flow passage 35BR, and is supplied to the container PB.

According to the studies of the present discloser, the entrainment of the surrounding air is most remarkable in a portion where the axis C10 of the discharge pipe 13 and the axis C30 of the moving nozzle 35 match. Therefore, as one preferred form, the pair of guide vanes 37 and 37 are disposed at positions that are line-symmetric with respect to the axis C30.

The pair of guide vanes 37 and 37 are preferably disposed such that upper ends thereof are positioned at the same position in the vertical direction V as the inlet port 35in. Since the position at which the upper ends of the guide vanes 37 and 37 are disposed is the highest position of the first flow passage 35B, the effect of preventing the entrainment of the air from the inside of at least the first flow passage 35B (35BL, 35BR) can be maximized.

The pair of guide vanes 37 and 37 are preferably disposed such that lower ends thereof reach a position below a position of 1/2 in the vertical direction V of the first flow passage 35B.

In a case where the interval between the pair of guide vanes 37 and 37 is excessively wide, the air is entrained in the second flow passage 37B, and in a case where the interval is excessively narrow, the smooth flow of the mist M may be hindered. Therefore, the specific interval of the guide vanes 37 and 37 can be determined in consideration of these factors.

[Behavior of Mist M by Moving Nozzle 35-3: Refer to FIG. 14]

With reference to FIG. 14, a situation in which the mist M is supplied to the moving nozzle 35-3 will be described.

In a case where the moving nozzle 35-3 has reached the discharge pipe 13 but the guide vanes 37 and 37 have not yet reached the discharge pipe 13 (T0), the mist M discharged from the discharge pipe 13 flows through the first-1 flow passage 35BR and is supplied to the container PB through the discharge port 35C. In this case, since the periphery of the mist M in the first-1 flow passage 35BR is relatively narrow, the entrainment of the air into the mist M is small. The same applies to the first-2 flow passage 35BL.

In a case where the guide vanes 37 and 37 reach the discharge pipe 13 (T1), the discharged mist M is supplied to the container PB through the second flow passage 37B between the guide vanes 37 and 37. At the position where the axis C10 of the discharge pipe 13 and the axis C30 of the moving nozzle 35-3 match, the surrounding air is most likely to be entrained into the mist M, but the guide vanes 37 and 37 can suppress the entrainment of the air. Therefore, since the decrease in temperature of the mist M passing through the second flow passage 37B is also suppressed, the reduction in the amount of the mist M supplied to the container PB can be suppressed.

In a case where the movement of the moving nozzle 35-3 further proceeds, the supply of the mist M transitions from the second flow passage 37B to the first-2 flow passage 35BL. In the first-2 flow passage 35BL, the entrainment of the air into the mist M may be small.

[Effects of Second Embodiment]

According to the moving nozzle 35-3 according to the second embodiment, since the guide vanes 37 and 37 are provided, the mist M can be reduced from entraining the surrounding air. As a result, the amount of the mist M supplied to the inside of the container PB by condensation due to the decrease in temperature of the mist M can be reduced.

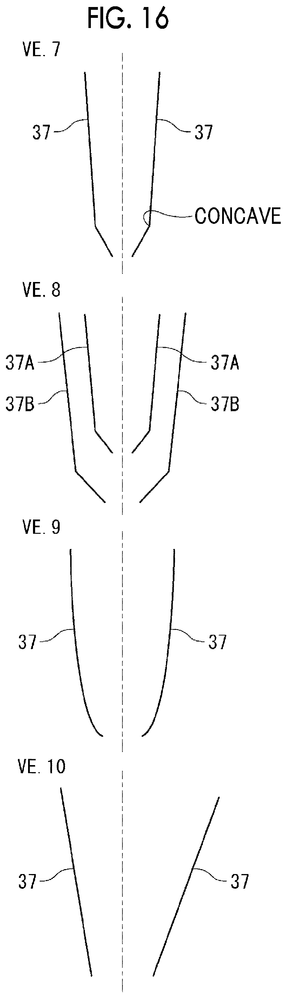

[Modification Examples: Refer to FIGS. 15 and 16]

Although the preferred embodiments of the present disclosure are described above, it is possible to select and adopt the configurations described in the embodiments or to appropriately change the configurations to other configurations.

FIG. 15 shows six modification examples (VE.1 to VE.6) of the moving nozzle. In the following, the modification examples will be described with differences from the moving nozzle 35-2.

In FIG. 15, the discharge port 35C provided in the moving nozzle 35-2 is omitted, and the outlet port 35out functions as the discharge port 35C.

The circumferential wall surface 35B5 and the circumferential wall surface 35B6 of the moving nozzle 35-2 are line-symmetric with respect to the axis C30, but the circumferential wall surfaces 35B1 and 35B2 of the moving nozzle 35-4 have an asymmetric relationship. In consideration of the fact that the moving nozzle 35-4 moves at a high speed, it is assumed that the circumferential wall surfaces 35B1 and 35B2 are asymmetric.

The circumferential wall surfaces 35B1 and 35B2 of the moving nozzle 35-2 are each composed of a flat surface, but as in the moving nozzle 35-6 and the moving nozzle 35-7, a bend may be provided in a part of the circumferential wall surfaces 35B1 and 35B2. The moving nozzle 35-6 has a concave bend, whereas the moving nozzle 35-7 has a convex bend.

In addition, as in the moving nozzle 35-8 and the moving nozzle 35-9, the circumferential wall surfaces 35B1 and 35B2 may have a curved surface in a part or all of the circumferential wall surfaces 35B1 and 35B2.

FIG. 16 shows three modification examples (VE.7 to VE.10) of the guide vane.

The guide vanes 37 and 37 are not limited to being composed of a flat surface as a whole, and a concave may be provided in the middle.

In addition, a plurality of guide vanes, specifically, two guide vanes 37A and 37B can be provided on one side.

Further, the guide vanes 37 and 37 each can also be composed of a curved surface.

Further, the guide vanes 37 and 37 can also be provided at positions that are asymmetric with respect to an axis indicated by a one-dot chain line.

[Supplementary Notes]

[1] A sterilization agent supply device (1) including:

a supply unit (10) including a discharge pipe (13) that supplies a sterilization agent downward in a vertical direction (V) at a fixed position; and
a transport unit (30) including a transport body (31) that moves along a transport path while holding a plurality of sterilization target objects (PB) by holding tools (GR), and a moving nozzle (35-1) that is provided on the transport body (31) corresponding to each of the plurality of holding tools (GR) and that moves in synchronization with the corresponding holding tool (GR), in which
the moving nozzle (35-1) includes a first flow passage (35B) that receives the sterilization agent upstream (US) and that allows the sterilization agent to flow toward the sterilization target object (PB) downstream (DS), and
in the first flow passage (35B), an opening area on an upstream (US) side is larger than an opening area on a downstream (DS) side.

[2] The sterilization agent supply device (1) according to [1], in which

the transport body (31) includes

a rotary table (31) that is rotationally driven by a drive source, and
the plurality of holding tools (GR) that are provided along the transport path connected to the rotary table (31) in a circumferential direction (CD), and

a plurality of the moving nozzles (35-1) correspond to the plurality of holding tools (GR), respectively, are arranged in a circumferential direction (CD) at the same position in a radial direction (RD) of the rotary table (31), and are attachably and detachably mounted on the rotary table (31).

[3] The sterilization agent supply device (1) according to [2], in which
in the first flow passage (35B), from the upstream (US) side to the downstream (DS) side,

an opening dimension in the circumferential direction (CD) is formed to decrease continuously or intermittently, and
an opening dimension in the radial direction (RD) is constant.

[4] The sterilization agent supply device (1) according to [2], in which
in the first flow passage (35B), a dimension in the circumferential direction (CD) is larger than a dimension in the radial direction (RD).

[5] The sterilization agent supply device (1) according to [2], in which
in the first flow passage (35B), an opening dimension (L35in) in the circumferential direction (CD) on the upstream (US) side is larger than a dimension (D) of an interval between adjacent first flow passages (35B).

[6] The sterilization agent supply device (1) according to any one of [2] to [5], in which
the first flow passage (35B) has a symmetric shape or an asymmetric shape in the circumferential direction (CD) with respect to an axis (C30) parallel to a vertical direction (V) of the moving nozzle (35-1).

[7] The sterilization agent supply device (1) according to any one of [2] to [6], in which

the moving nozzle includes (35-1) a first circumferential wall surface (35B1) and a second circumferential wall surface (35B2) that partition the first flow passage (35B) in the circumferential direction, and
the first circumferential wall surface (35B1) and the second circumferential wall surface (35B2) are inclined in the same direction in the circumferential direction (CD).

[8] The sterilization agent supply device (1) according to any one of [2] to [7], in which the first circumferential wall surface (35B1) and the second circumferential wall surface (35B2) are inclined forward in a movement direction of the moving nozzle.

[9] The sterilization agent supply device (1) according to any one of [2] to [8], in which

the second circumferential wall surface (35B2) is disposed behind the first circumferential wall surface (35B1) in a movement direction of the moving nozzle, and
in a case where inclinations of the first circumferential wall surface (35B1) and the second circumferential wall surface (35B2) with respect to a vertical direction (V) are denoted by $\theta1$ and $\theta2$, respectively, $\theta1 > \theta2$ is established.

[10] The sterilization agent supply device (1) according to any one of [2] to [9], in which

the moving nozzle (35-1) includes a second flow passage (37B) inside the first flow passage (35B), and
the second flow passage (37B) is partitioned from the first flow passage (35B) by a pair of guide vanes (37, 37) disposed at an interval in the circumferential direction (CD).

[11] The sterilization agent supply device (1) according to any one of [2] to [9], in which the pair of guide vanes (37, 37) are disposed at positions that are symmetric with respect to an axis (C30) parallel to a vertical direction (V) of the moving nozzle (35-1), and the interval narrows from the upstream (US) side to the downstream (DS) side.

[12] The sterilization agent supply device (1) according to any one of [2] to [11], in which a gap is preferably provided between the supply unit (10) and the moving nozzle (35-1) in the vertical direction (V).

[13] The sterilization agent supply device (1) according to any one of [2] to [12], in which the supply unit (10) is preferably provided with a restriction body (15) having a flat portion that closes an opening of the first flow passage (35B) on the upstream (US) side.

[14] A nozzle (35) provided between a discharge pipe (13) and a sterilization target object (PB), and that allows a sterilization agent supplied from the discharge pipe (13) to flow toward the sterilization target object, the nozzle (35) including:

a first flow passage (35B) that receives the sterilization agent upstream (US) and that allows the sterilization agent to flow toward the sterilization target object (PB) downstream (DS), in which
in the first flow passage (35B), an opening area on an upstream (US) side is larger than an opening area on a downstream (DS) side.

[15] The nozzle according to [14], in which

the moving nozzle (nozzle 35-1) includes a first circumferential wall surface (35B1) and a second circumferential wall surface (35B2) that partition the first flow passage in a first direction, and
the first circumferential wall surface (35B1) and the second circumferential wall surface (35B2) are inclined in the same direction in the first direction.

[16] A sterilization method of sterilizing a sterilization target object (PB) by supplying a mist-like sterilization agent discharged from a discharge pipe (13) to the sterilization target object (PB) through a nozzle (35), in which

the nozzle (35) includes a first flow passage (35B) that receives the sterilization agent upstream (US) and that allows the sterilization agent to flow toward the sterilization target object (PB) downstream (DS), and
in the first flow passage (35B), an opening area on an upstream (US) side is larger than an opening area on a downstream (DS) side.

[17] The sterilization method according to [16], in which the sterilization target object (PB) is a container that is filled with a beverage.

Reference Signs List

1: supply device
10: supply unit
11: mist generator
13: discharge pipe
13A: pipe body
13B: passage
13C: discharge port
15: restriction body
30: transport unit
31: rotary table
32: rotating electric machine
35-1, 35-2, 35-3, 35-4, 35-5, 35-6, 35-7, 35-8: moving nozzle

35A: nozzle housing
35B: first flow passage
35out: outlet port
35in: inlet port
35BL: first-2 flow passage
35BR: first-1 flow passage
35B1, 35B2, 35B5, 35B6: circumferential wall surface
35B3, 35B4, 35B7, 35B8: radial wall surface
35C: discharge port
37: guide vane
37B: second flow passage
C10, C30: axis
G: gap
M: mist
PB: container
N: mouth portion
RD: radial direction
CD: circumferential direction
MD: movement path
V: vertical direction
H: horizontal direction

## Claims

1. A sterilization agent supply device comprising:

   a supply unit including a discharge pipe that supplies a sterilization agent downward in a vertical direction at a fixed position; and
   a transport unit including a transport body that moves along a transport path while holding a plurality of sterilization target objects by holding tools, and a moving nozzle that is provided on the transport body corresponding to each of the plurality of holding tools and that moves in synchronization with the corresponding holding tool, wherein
   the moving nozzle includes a first flow passage that receives the sterilization agent upstream and that allows the sterilization agent to flow toward the sterilization target object downstream, and
   in the first flow passage, an opening area on an upstream side is larger than an opening area on a downstream side.

2. The sterilization agent supply device according to Claim 1, wherein

   the transport body includes

      a rotary table that is rotationally driven by a drive source, and
      the plurality of holding tools that are provided along the transport path connected to the rotary table in a circumferential direction, and

a plurality of the moving nozzles correspond to the plurality of holding tools, respectively, are arranged in a circumferential direction at the same position in a radial direction of the rotary table, and are attachably and detachably mounted on the rotary table.

3. The sterilization agent supply device according to Claim 2, wherein
   in the first flow passage, from the upstream side to the downstream side,

      an opening dimension in the circumferential direction decreases continuously or intermittently, and
      an opening dimension in the radial direction is constant or decreases.

4. The sterilization agent supply device according to Claim 2, wherein
   in the first flow passage, a dimension in the circumferential direction is larger than a dimension in the radial direction.

5. The sterilization agent supply device according to Claim 2, wherein
   in the first flow passage, an opening dimension in the circumferential direction on the upstream side is larger than a dimension of an interval between adjacent first flow passages.

6. The sterilization agent supply device according to Claim 2, wherein
   the first flow passage has a symmetric shape or an asymmetric shape in the circumferential direction with respect to an axis parallel to a vertical direction of the moving nozzle.

7. The sterilization agent supply device according to Claim 2, wherein

   the moving nozzle includes a first circumferential wall surface and a second circumferential wall surface that partition the first flow passage in the circumferential direction, and
   the first circumferential wall surface and the second circumferential wall surface are inclined in the same direction in the circumferential direction.

8. The sterilization agent supply device according to Claim 7, wherein
   the first circumferential wall surface and the second circumferential wall surface are inclined forward in a movement direction of the moving nozzle.

9. The sterilization agent supply device according to Claim 7 or 8, wherein

the second circumferential wall surface is disposed behind the first circumferential wall surface in a movement direction of the moving nozzle, and
in a case where inclinations of the first circumferential wall surface and the second circumferential wall surface with respect to a vertical direction are denoted by θ1 and θ2, respectively, θ1 > θ2 is established.

10. The sterilization agent supply device according to Claim 2, wherein

the moving nozzle includes a second flow passage inside the first flow passage, and
the second flow passage is partitioned from the first flow passage by a pair of guide vanes disposed at an interval in the circumferential direction.

11. The sterilization agent supply device according to Claim 10, wherein
the pair of guide vanes are disposed at positions that are symmetric or asymmetric with respect to an axis parallel to a vertical direction of the moving nozzle, and the interval narrows from the upstream side to the downstream side.

12. The supply device according to Claim 1, wherein
a gap is provided between the supply unit and the moving nozzle in the vertical direction.

13. The supply device according to Claim 1, wherein
the supply unit is provided with a restriction body having a flat portion that closes an opening of the first flow passage on the upstream side.

14. A nozzle provided between a discharge pipe and a sterilization target object, and that allows a sterilization agent supplied from the discharge pipe to flow toward the sterilization target object, the nozzle comprising:

a first flow passage that receives the sterilization agent upstream and that allows the sterilization agent to flow toward the sterilization target object downstream, wherein
in the first flow passage, an opening area on an upstream side is larger than an opening area on a downstream side.

15. The nozzle according to Claim 14, wherein

the moving nozzle includes a first circumferential wall surface and a second circumferential wall surface that partition the first flow passage in a first direction, and
the first circumferential wall surface and the second circumferential wall surface are inclined in the same direction in the first direction.

16. A sterilization method of sterilizing a sterilization target object by supplying a sterilization agent discharged from a discharge pipe to the sterilization target object through a nozzle, wherein

the nozzle includes a first flow passage that receives the sterilization agent upstream and that allows the sterilization agent to flow toward the sterilization target object downstream, and
in the first flow passage, an opening area on an upstream side is larger than an opening area on a downstream side.

17. The sterilization method according to Claim 16, wherein
the sterilization target object is a container that is filled with a beverage.

# FIG. 1

# FIG. 2

# FIG. 3

# FIG. 4

# FIG. 5

# FIG. 6

## FIG. 7

$$\theta1 > \theta2$$
$$55° \leqq \theta1 \leqq 85°$$
$$20° \leqq \theta2 \leqq 50°$$

## FIG. 8

$$W35in \geqq W35out$$

## FIG. 9

35-1

13C

MD

35-1

13C

MD, CD

35-1

13C

MD, CD

# FIG. 10

# FIG. 11

CD

T0

T1

T2

T3

T4

# FIG. 12

# FIG. 13

## FIG. 14

# FIG. 15

VE. 1

35B6      35B5

35B

35-4

VE. 2

35B6   C30    35B5

$\theta 11$   $\theta 21$

35-5

VE. 3

35B6      35B5

CONCAVE   35B

35-6

VE. 4

35B6      35B5

CONVEX   35B

35-7

VE. 5

35B6      35B5

35-8

VE. 6

35B6      35B5

35-9

## FIG. 16

VE. 7

37 — 37

CONCAVE

VE. 8

37A — 37A

37B — 37B

VE. 9

37 — 37

VE. 10

37 — 37

## INTERNATIONAL SEARCH REPORT

| International application No. |
| --- |
| **PCT/JP2024/016655** |

| A. | CLASSIFICATION OF SUBJECT MATTER |
| --- | --- |

***B65B 55/10***(2006.01)i; ***B65B 55/04***(2006.01)i
FI:    B65B55/10 A; B65B55/10 F; B65B55/04 C

According to International Patent Classification (IPC) or to both national classification and IPC

| B. | FIELDS SEARCHED |
| --- | --- |

Minimum documentation searched (classification system followed by classification symbols)

B65B55/10; B65B55/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | JP 2020-138802 A (DAI NIPPON PRINTING CO., LTD.) 03 September 2020 (2020-09-03) paragraphs [0038]-[0049], [0054]-[0064], fig. 2, 5A-7 | 1-6, 12-14, 16-17 |
| A | | 7-11, 15 |
| A | JP 2022-96392 A (DAI NIPPON PRINTING CO., LTD.) 29 June 2022 (2022-06-29) | 1-17 |
| A | JP 2020-125117 A (MITSUBISHI HEAVY IND MACH SYSTEMS LTD.) 20 August 2020 (2020-08-20) | 1-17 |

| ☐ Further documents are listed in the continuation of Box C. | ☑ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **02 July 2024** | **16 July 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

EP 4 763 724 A1

## INTERNATIONAL SEARCH REPORT
### Information on patent family members

| Patent document cited in search report | | | Publication date (day/month/year) | Patent family member(s) | | | Publication date (day/month/year) |
|---|---|---|---|---|---|---|---|
| JP | 2020-138802 | A | 03 September 2020 | US | 2022/0127125 | A1 | |
| | | | | paragraphs [0051]-[0062], [0067]-[0077], fig. 2, 5A-7 | | | |
| | | | | WO | 2020/179521 | A1 | |
| | | | | EP | 3932853 | A1 | |
| | | | | CN | 113382951 | A | |
| JP | 2022-96392 | A | 29 June 2022 | WO | 2022/130868 | A1 | |
| | | | | CN | 116615378 | A | |
| JP | 2020-125117 | A | 20 August 2020 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP 2015157651 A